# EUROPÄISCHE PATENTSCHRIFT

(11) **EP 4 110 428 B1**
(45) Veröffentlichungstag und Bekanntmachung des Hinweises auf die Patenterteilung: **06.05.2026**
(21) Anmeldenummer: 21717425.9
(22) Anmeldetag: 07.04.2021
(51) Int. Cl.: A61M 5/14

(54) **VERFAHREN ZUR HERSTELLUNG EINER TROPFKAMMER, TROPFKAMMER, INFUSIONS- ODER TRANSFUSIONSSYSTEM**
METHOD FOR PRODUCING A DRIP CHAMBER, DRIP CHAMBER, INFUSION SYSTEM OR TRANSFUSION SYSTEM
PROCÉDÉ DE FABRICATION D'UNE CHAMBRE COMPTE-GOUTTES, CHAMBRE COMPTE-GOUTTES, SYSTÈME DE PERFUSION OU SYSTÈME DE TRANSFUSION

(30) Priorität: 09.04.2020 DE 102020204611
(43) Veröffentlichungstag der Anmeldung: 04.01.2023
(73) Patentinhaber: B. Braun Melsungen AG, 34212 Melsungen (DE)
(72) Erfinder: SCHLITT, Christof, 34621 Frielendorf (DE); SEIDEL, Gerrit, 34121 Kassel (DE)
(74) Vertreter: DREISS Patentanwälte PartG mbB
(86) Internationale Anmeldenummer: PCT/EP2021/059109
(87) Internationale Veröffentlichungsnummer: WO 2021/204904

(56) Entgegenhaltungen:
- EP-A1- 0 355 795
- DE-A1- 19 931 092
- DE-A1- 2 431 946
- GB-A- 768 689
- US-A- 4 395 260
- US-A1- 2017 151 385

## Beschreibung

Zu therapeutischen Zwecken werden in der Human- und Veterinärmedizin Infusionen und Transfusionen durchgeführt. Beispielsweise dienen Infusionen der Verabreichung von flüssigen Arzneimitteln (Wirkstofflösungen etc.). Unter einem Infusions- oder Transfusionssystem wird ein System verstanden, mit dem die Verabreichung einer medizinischen Infusion oder die Durchführung einer medizinischen Transfusion vorgenommen werden kann. Insbesondere kann es sich um ein Infusionsset (auch als "Infusionsbesteck" bezeichnet) handeln.

Ein Infusions- oder Transfusionssystem weist in der Regel einen Schlauch und eine Tropfkammer auf. Das Infusions- oder Transfusionssystem kann optional weitere Komponenten umfassen, beispielsweise einen Durchflussregler zur Kontrolle der Fließgeschwindigkeit der Flüssigkeit wie etwa eine Rollenklemme. Die im Rahmen einer Infusion oder Transfusion zu verabreichende Flüssigkeit wird in einem Behälter bereitgestellt. Bei dem Behälter kann sich beispielsweise um eine Infusionsflasche, einen Infusionsbeutel, eine Blutkonserve etc. handeln.

Die Tropfkammer wird über einen Behälteranschluss mit dem Behälter verbunden, sodass die Flüssigkeit aus dem Behälter in die Tropfkammer gelangen kann. Bei dem Behälteranschluss kann es sich beispielsweise um eine Stechvorrichtung wie etwa einen hohlen Dorn handeln, mit dem ein den Behälter verschließendes Septum durchstochen werden kann und der in seinem Inneren typischerweise mehrere Kanäle aufweist. Eine derartige Stechvorrichtung wird allgemein als "Spike" bezeichnet. Es sind daneben weitere Systeme bekannt, um die Tropfkammer mit dem Behälter zu verbinden, beispielsweise Kupplungssysteme, welche es nicht zulassen, dass die Tropfkammer und der Behälter, nachdem diese miteinander verbunden worden sind, wieder getrennt werden können. Die Tropfkammer steht in Fluidverbindung mit einem Ende des Schlauchs, sodass Flüssigkeit aus der Tropfkammer in den Schlauch eintreten kann. In diesem Sinne ist die Tropfkammer ein Flüssigkeitsüberleitungssystem, da die Flüssigkeit aus dem Behälter durch die Tropfkammer in den Schlauch übergeleitet wird. Der Schlauch weist an einem anderen Ende einen Anschluss für einen Patientenzugang (z.B. Venenkanüle oder Venenkatheter) auf. Der Patientenzugang kann optional auch als Teil des Infusionssets betrachtet werden.

Die Tropfkammer stellt, wie beschrieben, die Verbindung zwischen Schlauch und dem Behälter her. In der Regel sind die Vorrichtungen, die die Belüftung des Behälters gewährleisten, in die Tropfkammer integriert. Dazu weist die Tropfkammer üblicherweise eine Belüftungsvorrichtung mit einem manuell zu bedienenden oder einem automatischen Belüftungsventil auf. Ein Belüftungskanal ermöglicht den Luftzutritt in den Behälter. Damit keine Kontaminationen auftreten, wenn Luft aus der Umgebung durch das Belüftungsventil strömt, weist die Belüftungsvorrichtung üblicherweise ein Belüftungsfilter auf. Um zuverlässig zu verhindern, dass Flüssigkeit zum Belüftungsfilter vordringt, diesen durchtränkt und so für Luft undurchdringbar macht, ist am Belüftungskanal häufig ein Verschlussmechanismus vorgesehen. Im Stand der Technik sind unterschiedliche Ausführungsformen der Belüftungsvorrichtung auf Basis verschiedener Ventiltypen sowie mit und ohne Belüftungsfilter und Verschlussmechanismus bekannt, beispielsweise manuelle Belüftungsvorrichtungen, die über eine manuell zu betätigende Klappe als Belüftungsventil verfügen, sowie automatische Belüftungsventile, die ein Rückschlagventil als Belüftungsventil aufweisen. Vorliegende Erfindung ist mit manuellen und automatischen Belüftungsvorrichtungen kompatibel.

Um beispielsweise eine Infusion zu beginnen, d.h. Flüssigkeit durch den Schlauch fließen zu lassen, wird zuerst die Tropfkammer über den Behälteranschluss mit dem Behälter verbunden. Dann muss das Fließen der Flüssigkeit initiiert werden. Dies erfolgt in der Regel dadurch, dass die zumindest in ihrem unteren Bereich aus einem elastischen Material bestehende Tropfkammer von Hand (beispielsweise zwischen Daumen und einem Finger) zusammengedrückt wird. Dadurch wird beispielsweise bei Verwendung einer Tropfkammer mit einer Stechvorrichtung (Spike) Luft durch einen der Kanäle der Stechvorrichtung in den Infusionsbehälter gepumpt. Durch den im Infusionsbehälter entstehenden Überdruck wird das Fließen der Flüssigkeit initiiert, d.h. die Flüssigkeit beginnt, in das Innere der Tropfkammer hineinzutropfen. Das Belüftungsventil sorgt für den Druckausgleich, der dafür erforderlich ist, dass die Flüssigkeit weiter in die Tropfkammer hineintropfen kann, indem sie den Lufteintritt durch einen der Kanäle der Stechvorrichtung (Belüftungskanal) gestattet, während die Flüssigkeit durch einen anderen der Kanäle aus dem Behälter in die Tropfkammer fließt. Die Tropfkammer nimmt nach der Kraftanwendung zum Initiieren des Fließens der Flüssigkeit ihre ursprüngliche Form wieder an. Alternativ oder zusätzlich kann auch auf den Behälter ein Druck ausgeübt werden. Da dies aber nur im Fall von flexiblen Behältern möglich ist, weisen die meisten der am Markt erhältlichen Tropfkammern einen flexiblen und somit pumpbaren unteren Abschnitt auf.

Im Inneren der Tropfkammer ein Tropfenformer vorgesehen, der bewirkt, dass die Flüssigkeit aus dem Behälter in Form von Tropfen normierter Größe in die Tropfkammer eintritt. Bei der Schwerkraftinfusion wird die Flussrate durch die Tropffrequenz, d.h. die Tropfenzahl pro Zeiteinheit, eingestellt. Die Tropffrequenz wird dabei vom Bediener durch visuelle Beobachtung des Tropfvorgangs festgestellt. Für manche Anwendungen, bei denen eine Infusionspumpe zum Fördern der Flüssigkeit verwendet wird, kommt ein Tropfensensor zum Einsatz, mit dem die Tropffrequenz optisch gemessen wird. Auf Grundlage der so gemessenen Tropffrequenz wird die Infusionspumpe gesteuert. Sowohl für die visuelle Beobachtung als auch für die optische Sensorik muss die Wand der Tropfkammer zumindest im oberen Abschnitt, in dem der Tropfenformer angeordnet ist, ausreichend durchsichtig sein.

Die Dimensionen einer Tropfkammer sind teilweise in den einschlägigen Normen (z.B. DIN ISO 8536-4, Teil 4) vorgegeben.

Manche der am Markt erhältlichen Behälter für Infusionsflüssigkeiten ermöglichen ein Zudosieren von weiteren Arzneimitteln (sogenanntes "Pharmacy Admixture"). Das Zudosieren erfolgt beispielsweise durch Zuspritzen über am Behälter vorgesehenen Port. Üblicherweise ist dieser Port an einer Stelle des Behälters in der Nähe der Position des Ports, über den der Behälteranschluss der Tropfkammer mit dem Behälter verbunden ist, angeordnet. Die Tropfkammer muss dann so gestaltet sein, dass der Port zum Zudosieren für den Bediener leicht zugänglich ist, wenn der Behälteranschluss der Tropfkammer mit dem Behälter verbunden ist. Insbesondere muss die Tropfkammer dafür ausreichend langgestreckt und entsprechend schmal geformt sein.

Die im Stand der Technik bekannten und am Markt erhältlichen Tropfkammern weisen üblicherweise ein kurzes und nicht hochdurchsichtiges Oberteil und ein pumpbares Unterteil auf. Manche der am Markt erhältlichen Tropfkammern verfügen zusätzlich über einen Griffring, der in dem Bereich der Außenwand der Tropfkammer ausgebildet ist. Der Griffring bildet eine Wulst, was die Handhabbarkeit der Tropfkammer verbessert, da der Bediener die Tropfkammer an der Wulst sicher mit der Hand fassen kann. Dieser Griffring besteht typischerweise aus einem opaken Kunststoff. Er wird mittels eines Spritzgießverfahrens an der Außenfläche der Tropfkammer angeformt.

Je nachdem, ob ein Griffring vorhanden ist oder nicht, wird von einem "dreiteiligen Aufbau" oder "zweiteiligen Aufbau" gesprochen.

Der Griffring wird herkömmlich in einem separaten Schritt auf der Außenfläche der Tropfkammer ausgebildet. Dies birgt Nachteile, insbesondere besteht ein sehr hoher Aufwand, die herkömmlich mittels des aufwendigen Spritzgießverfahrens hergestellten dreiteiligen Tropfkammern bereitzustellen. Das Spritzgießverfahren, das herkömmlich zum Anformen des Griffrings verwendet wird, ist apparativ sehr aufwendig.

Auf den Griffring zu verzichten, würde umgekehrt bedeuten, dass auf die oben beschriebenen ergonomischen Vorteile des Griffrings verzichtet wird. Außerdem bringt der Griffring den Vorteil mit sich, dass er die Fügestelle der Tropfkammer kaschiert, sodass sie in der Wahrnehmung des Anwenders keine von außen sichtbaren Fügestellen aufweist. Darüber hinaus kann das Material des Griffrings so gewählt werden, dass es besonders griffig ist, was die Handhabbarkeit noch weiter verbessert. Ferner ist es möglich, das Material des Griffrings einzufärben. Durch eine spezielle Farbgebung ist es möglich, eine Tropfkammer eines bestimmten Typs oder eines bestimmten Herstellers im Sinne einer Kennzeichnung zu individualisieren. Auf diese Weise können Verwechslungen im klinischen Betrieb vermieden werden.

Bei den im Stand der Technik bekannten und am Markt erhältlichen Tropfkammern sind Oberteil und Unterteil direkt miteinander verbunden, beispielsweise verklebt oder verschweißt. Das bringt zusätzlich zum hohen Aufwand im Fall der herkömmlichen Tropfkammern erhebliche Einschränkungen bei der Wahl der für Oberteil und Unterteil verwendeten Materialien mit sich, weil diese Materialien so auszuwählen sind, dass sie sich sicher und auf möglichst einfache Art und Weise aneinander fügen lassen. Mit anderen Worten müssen die Materialien hinsichtlich der Verbindbarkeit zueinander kompatibel sein. Daneben sollen die Materialien aber auch den an eine Tropfkammer gestellten Anforderungen genügen, beispielsweise was die Transparenz des Oberteils und die für das Pumpen erforderliche Elastizität des Unterteils betrifft.

US 2017 /0151385 A1 offenbart bereits eine Tropfkammer zur Verabreichung einer medizinischen Flüssigkeit. Die Tropfkammer umfasst ein Kammerelement, das eine Kammer bildet, einen Einlass zum Einlassen einer medizinischen Flüssigkeit in die Kammer und einen Verbinder, der mit dem Kammerelement an einem Auslass der Tropfkammer verbunden ist. Dabei ist ein Anschlussstück einstückig am Kammerelement durch Spritzgießen ausgebildet. Das Kammerelement ist aus einem ersten Material und das Anschlussstück aus einem zweiten Material gebildet ist, das sich von dem ersten Material unterscheidet.

US 4,395,260, DE19931092A1 und GB768689A offenbaren weitere mehrteilige Tropfkammern.

In EP 0 355 795 A1 ist ein Adapter zum Anschließen von enteralen Überleitungsgeräten wie einer Tropfkammer an Flaschen beschrieben. Der Adapter weist zwei koaxial zueinander angeordnete Kappen mit verschiedenen Öffnungsdurchmessern auf, die jeweils auf eine zugeordnete Flaschenöffnung aufsetzbar sind.

Ausgehend von der obengenannten Situation besteht eine Aufgabe der Erfindung darin, ein verbessertes System bzw. Verfahren für eine Tropfkammer für ein Infusions- oder Transfusionssystem, eine verbesserte Tropfkammer für ein Infusions- oder Transfusionssystem und ein verbessertes Infusions- oder Transfusionssystem bereitzustellen.

Diese Aufgabe wird gelöst durch ein Verfahren gemäß Anspruch 1, eine Tropfkammer gemäß Anspruch 2, und ein Infusions- oder Transfusionssystem gemäß Anspruch 11. Vorteilhafte Ausgestaltungen der Erfindung ergeben sich insbesondere aus den Unteransprüchen. Dabei können die in Unteransprüchen und nachfolgender Beschreibung des Gegenstands zu einem unabhängigen Anspruch angeführten Merkmale auch zur vorteilhaften Ausgestaltung des Gegenstands zu einem anderen Anspruch derselben oder einer anderen Anspruchskategorie verwendet werden.

Aufgrund der Unmöglichkeit oder Schwierigkeit, das aufwendige Spritzgießverfahren zum Anformen des Griffrings auf die Ausbildung eines anderen Typs des Griffrings aus einem anderen Material oder mit einer anderen Form umzustellen, hat es sich wegen der genannten Anforderung der Materialkompatibilität als sehr aufwendig erwiesen, verschiedene Tropfkammertypen rational und wirtschaftlich nach dem Baukastenprinzip auszulegen. Unter dem Begriff "Baukastensystem" wird dabei ein Konzept für die Gestaltung bzw. das Design sowie für die Fertigung verstanden, bei dem verschieden ausgelegte Oberteile und/oder verschieden ausgelegte Unterteile und/oder verschieden ausgelegte Mittelteile zum Einsatz kommen, jedoch jedes Oberteil, jedes Unterteil und jedes Mittelteil für die Herstellung einer Tropfkammer verwendet werden kann. Auf diese Weise lassen sich Tropfkammern verschiedener Typen auf effektive Weise herstellen. Das betrifft etwa verschiedene Oberteile mit verschiedenen Tropfenformern und/oder verschiedenen Typen von Belüftungsvorrichtungen. Das betrifft ferner etwa verschiedene Oberteile mit unterschiedlichen Qualitäten der Durchsichtigkeit oder der optischen Brillanz. Beispielsweise kann eine niedrigere Qualität der Durchsichtigkeit für die visuelle Beobachtung des Tropfens ausreichend sein, während eine höhere Qualität der Durchsichtigkeit Voraussetzung für die Verwendung optischer Tropfensensoren sein kann. Das betrifft auch verschiedene Unterteile, etwa weichere, die sich leichter pumpen lassen, und steifere, die stabiler sind. Das betrifft auch ganz allgemein verschiedene Größen von Oberteilen und Unterteilen.

Gemäß dem erfindungsgemäßen Verfahren wird Schritt D so ausgeführt, dass das Oberteil und das Unterteil nicht direkt miteinander verbunden sind, sondern über das Mittelteil. Das heißt, dass das Oberteil und das Unterteil nicht aneinandergefügt sind, sondern durch das Mittelteil zusammengehalten werden. Dabei ist es möglich aber nicht erforderlich, dass das Oberteil und das Unterteil in der fertig hergestellten Tropfkammer einander berühren. Gemäß dieser Ausführungsform können die Fügeschritte in beliebiger Reihenfolge ausgeführt werden. Es ist demnach möglich zuerst das Oberteil und dann das Unterteil oder zuerst das Unterteil und dann das Oberteil mit dem Mittelteil zu verbinden. Es ist darüber hinaus auch möglich, das Oberteil und das Unterteil zumindest teilweise gleichzeitig mit dem Mittelteil zu verbinden. Für die einzelnen Schritte A, B, C und D des Verfahrens sind verschiedene Reihenfolgen möglich, solange sichergestellt ist, dass die miteinander zu verbindenden Teile rechtzeitig bereitgestellt sind, um miteinander verbunden werden zu können.

Beim erfindungsgemäßen Verfahren wird kein Mittelteil in Gestalt eines Griffrings mittels eines aufwendingen Spritzgießverfahrens an der Außenfläche der Tropfkammer angeformt. Anstatt dessen ist ein vorgefertigtes Mittelteil vorgegeben. Dieses Mittelteil muss lediglich mit dem Oberteil und/oder dem Unterteil verbunden werden. Dies verringert den fertigungstechnischen Aufwand der Herstellung einer dreiteiligen Tropfkammer maßgeblich. Die Verringerung des fertigungstechnischen Aufwands ist vorliegend besonders sinnvoll, da es sich bei Infusionssystemen um Massenartikel handelt, die in großer Stückzahl hergestellt werden.

Vorteilhaft an dem erfindungsgemäßen Verfahren ist ferner, dass das Verfahren sehr einfach auf die Herstellung einer Tropfkammer mit einem anderen Typ des Griffrings umgestellt werden kann. Dazu muss lediglich in Schritt C ein anderes Mittelteil bereitgestellt werden. Das aufwendige Umstellen einer Anlage, mit der der Griffring durch ein Spritzgießverfahren angeformt wird, entfällt.

Im erfindungsgemäßen Verfahren werden die Verbindungen der einzelnen Teile zumindest teilweise mittels Lösungsmittel-Fügen hergestellt. Dadurch lassen sich nicht nur weiter fertigungstechnische Vorteile im Sinne einer Vereinfachung der Herstellung erzielen, sondern auch eine Verbesserung des hergestellten Erzeugnisses.

Beim Lösungsmittel-Fügen wird zumindest eines der miteinander zu verbindenden Fügeteile mit einem geeigneten organischen Lösungsmittel behandelt. Anschließend werden die Fügeteile aneinandergefügt und bevorzugt gegeneinander in Form einer Scherbewegung bewegt. Nach einer nicht einschränkenden Theorie verschlaufen die Moleküle der Fügeteile miteinander, sodass an der Fügestelle eine Verbindung entsteht. Das Verfahren des Lösungsmittel-Fügens lässt sich insbesondere im Zuge einer automatisierten Fertigung rasch und einfach umsetzen. Somit handelt es sich beim Lösungsmittel-Fügen um eine kostengünstige Fügemethode. Das Lösungsmittel-Fügen wird auch als "Lösungsmittel-Kleben" bezeichnet, obwohl es sich nicht um ein Klebeverfahren im eigentlichen Sinn handelt, bei dem ein Klebstoff eingesetzt wird, der die Verklebung der Fügestellen herstellt.

Die erfindungsgemäße Tropfkammer, die insbesondere mit dem erfindungsgemäßen Verfahren erhältlich ist, umfasst das Oberteil, das Unterteil und das Mittelteil. Das Mittelteil ist erfindungsgemäß vorgefertigt, d.h. ein nachträgliches Aufbringen durch Spritzguss entfällt und wird vermieden. Das vorgefertigte Mittelteil ist in einer ersten Ausführungsform (a) so eingerichtet, dass es eine jeweils unmittelbare fluiddichte Verbindung mit dem Oberteil und mit dem Unterteil schafft, indem ein unterer Endbereich des Oberteils und ein oberer Endbereich des Unterteils jeweils mit dem Mittelteil gefügt sind. In dieser Ausführung reicht es aus, dass sich diese Ränder lediglich kontaktieren aber nicht miteinander gefügt sind; die Ränder können einfach aneinander gegenüberliegen oder sogar lediglich mit einem dazwischenliegenden Spalt einander zugewandt sein.

In einer zweiten Ausführungsform (b) ist das vorgefertigte Mittelteil so eingerichtet, dass es eine Überbrückung eines unteren Endbereichs des Oberteils und eines oberen Endbereichs des Unterteils bereitstellt, wobei der untere Rand des Oberteils und der obere Rand des Unterteils direkt miteinander gefügt sind und das Mittelteil die Überbrückung ohne Spritzguss bereitstellt, gegebenenfalls aber mit einem unteren Endbereich des Oberteils und/oder mit einem oberen Endbereich des Unterteils gefügt ist. Wenn das Oberteil und das Unterteil in dieser Ausführungsform (b) nicht mit dem vorgefertigten Mittelteil gefügt sind, kann das Mittelteil einfach in einer Nut gehalten werden, die durch einen geringeren Durchmesser im Übergang zwischen unterem Endbereich des Oberteils und oberem Endbereich des Unterteils gebildet ist.

Fluiddichte Fügestellen sind aufgrund des vorgefertigten Mittelteils, welches eine jeweils unmittelbare fluiddichte Verbindung mit dem Oberteil und mit dem Unterteil bereitstellt oder welches mittelbar eine Überbrückung von Oberteil mit Unterteil bereitstellt, gewährleistet. Ferner sind dadurch, dass der Einsatz eines vorgefertigten Mittelteils ein Lösungsmittel-Fügen oder ein Schweißen, insbesondere ein Ultraschallschweißen an den jeweiligen Fügestellen mit dem vorgefertigten Mittelteil ermöglicht, sowohl eine Fluiddichtheit als auch eine Überbrückung der Nahtstelle zwischen Ober- und Unterteil sicher gewährleistet.

Bei der Tropfkammer gemäß einer speziellen Ausführungsform der Erfindung handelt es sich um eine Tropfkammer für ein Infusions- oder Transfusionssystem, umfassend ein Oberteil mit einem ersten Anschluss, welcher mit einem Behälter verbunden ist oder welcher bestimmt ist, mit einem Behälter verbunden zu werden, ein Unterteil mit einem zweiten Anschluss, welcher mit einem Schlauch verbunden ist oder welcher bestimmt ist, mit einem Schlauch verbunden zu werden, und ein Mittelteil. Das Oberteil und das Unterteil sind jeweils so mit dem Mittelteil verbunden, dass das Oberteil und das Unterteil über das Mittelteil fluiddicht miteinander verbunden sind.

Indem gemäß dieser Ausführungsform das Oberteil und das Unterteil nicht direkt sondern über das Mittelteil fluiddicht miteinander verbunden sind, besteht hinsichtlich der Auswahl der Materialien für das Oberteil und das Unterteil eine große Freiheit. So können die Materialien jeweils hinsichtlich der Funktionen des Oberteils und des Unterteils optimal ausgewählt werden. Beispielsweise kann für das Oberteil ein Kunststoff mit hoher Brillanz gewählt werden, während für das Unterteil ein Kunststoff gewählt wird, der für das Pumpen optimale Eigenschaften aufweist und zudem für Transport und Lagerung ausreichend robust ist. Dabei müssen die Materialien nicht so aufeinander abgestimmt sein, dass sie einfach und sicher direkt aneinander gefügt werden können. Es muss lediglich für das Mittelteil ein Material gewählt werden, welches sowohl an das Oberteil als auch an das Unterteil gefügt werden kann.

Indem gemäß dieser Ausführungsform das Oberteil und das Unterteil nicht direkt sondern über das Mittelteil fluiddicht miteinander verbunden sind, besteht hinsichtlich der Auswahl der Fügetechniken zur Verbindung der einzelnen Teile eine große Freiheit. So ist es beispielsweise möglich, eine bestimmte Klebetechnik auch dann zu verwenden, wenn Oberteil und Unterteil mit dieser Technik nicht oder nur unter großem Aufwand verbunden werden können. Es muss lediglich für das Mittelteil ein Material gewählt werden, welches mittels dieser Technik sowohl an das Oberteil als auch an das Unterteil gefügt werden kann.

Da das Mittelteil anders als der im Stand der Technik bekannte Griffring nicht als separates Element in einem separaten Fertigungsschritt angebracht werden muss, ist die Herstellung der erfindungsgemäßen Tropfkammern besonders vorteilhaft. Dies gilt insbesondere deshalb, weil ein aufwendiges Spritzgießverfahren vermieden werden kann.

Gemäß dieser Ausführungsform lassen sich somit die Vorteile des dreiteiligen Aufbaus der Tropfkammer und damit die ergonomischen und sonstigen Vorteile eines Griffrings optimal realisieren. Dazu zählen insbesondere die optimal ergonomischen und intuitiven Handhabbarkeit, die Möglichkeit der Kennzeichnung verschiedener Tropfkammertypen oder auf andere Weise voneinander verschiedener Produkte, die verbesserte Möglichkeit einer Baukastenfertigung und daher die einfachere und ökonomisch günstigere Implementierung weiterer oder anderer Funktionen.

Bei dem erfindungsgemäßen Infusions- oder Transfusionssystem handelt es sich um ein Infusions- oder Transfusionssystem, welches eine erfindungsgemäße Tropfkammer umfasst. Damit wird ein Infusions- oder Transfusionssystem bereitgestellt, das über die vorteilhaften Eigenschaften der erfindungsgemäßen Tropfkammer verfügt.

Weitere Merkmale, Zweckmäßigkeiten und Vorteile der Erfindung werden nachfolgend anhand von exemplarischen Ausführungsbeispielen unter Bezugnahme auf die angeschlossenen Zeichnungsfiguren beschrieben.
Fig. 1 zeigt ein Ablaufdiagramm eines Ausführungsbeispiels des erfindungsgemäßen Verfahrens.
Fig. 2 zeigt eine schematische perspektivische Ansicht einer Tropfkammer gemäß einem ersten Ausführungsbeispiel.
Fig. 3 zeigt eine schematische Ansicht der Tropfkammer gemäß dem ersten Ausführungsbeispiel in einer Explosionsdarstellung.
Fig. 4 zeigt eine schematische Ansicht der Tropfkammer gemäß einem zweiten Ausführungsbeispiel in einer Explosionsdarstellung.

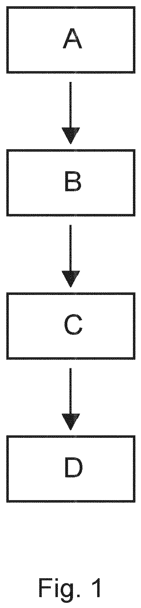

In Fig. 1 ist ein Ablaufdiagramm eines Ausführungsbeispiels des erfindungsgemäßen Verfahrens zur Herstellung einer Tropfkammer dargestellt. Die einzelnen Teile der Tropfkammer werden dabei weiter unten im Zuge der Beschreibung von exemplarischen Ausführungsbeispielen der Tropfkammer näher beschrieben. Das erfindungsgemäße Verfahren ist nicht auf die Herstellung von Tropfkammern gemäß dieser Ausführungsbeispiele beschränkt.

Im Schritt A wird ein Oberteil bereitgestellt. Das Oberteil weist einen ersten Anschluss auf, welcher bestimmt ist, mit einem Behälter verbunden zu werden.

Im nachfolgenden Schritt B wird ein Unterteil bereitgestellt. Das Unterteil weist einen zweiten Anschluss auf, welcher mit einem Schlauch verbunden ist oder welcher bestimmt ist, mit einem Schlauch verbunden zu werden.

Im nachfolgenden Schritt C wird ein Mittelteil bereitgestellt. Das Mittelteil stellt in der fertig hergestellten Tropfkammer einen Griffring bereit, d.h. eine an der Außenseite der Tropfkammer angeordnete erhabene Struktur.

Im nachfolgenden Schritt D erfolgt das Verbinden des Oberteils, des Unterteils und des Mittelteils.

Gemäß einer ersten Ausführungsform des erfindungsgemäßen Verfahrens wird Schritt D so ausgeführt, dass das Oberteil und das Unterteil nicht direkt miteinander verbunden sind, sondern über das Mittelteil. Das heißt, dass das Oberteil und das Unterteil nicht aneinandergefügt sind, sondern durch das Mittelteil zusammengehalten werden. Gemäß dieser Ausführungsform können die Fügeschritte in beliebiger Reihenfolge ausgeführt werden. Es ist demnach möglich zuerst das Oberteil und dann das Unterteil oder zuerst das Unterteil und dann das Oberteil mit dem Mittelteil zu verbinden. Es ist darüber hinaus auch möglich, das Oberteil und das Unterteil zumindest teilweise gleichzeitig mit dem Mittelteil zu verbinden.

Gemäß einer andern Ausführungsform des erfindungsgemäßen Verfahrens wird Schritt D so ausgeführt, dass das Oberteil und das Unterteil direkt miteinander verbunden sind. Das Mittelteil ist dann nicht erforderlich, um das Oberteil und das Unterteil zusammenzuhalten. Anstatt dessen wird das Mittelteil außen an der aus Oberteil und Unterteil bestehenden Holkörper angeordnet. Gemäß dieser Ausführungsform können die Fügeschritte in beliebiger Reihenfolge ausgeführt werden. Es ist demnach möglich zuerst das Oberteil mit dem Mittelteil und dann das Unterteil mit dem Oberteil oder zuerst das Unterteil mit dem Mittelteil und dann das Oberteil mit dem Unterteil zu verbinden. Es ist ferner möglich, zuerst das Oberteil und das Unterteil miteinander zu verbinden und dann das Mittelteil mit dem Oberteil, dem Unterteil oder mit Oberteil und Unterteil zu verbinden. Es ist darüber hinaus auch möglich, alle Fügeschritte zumindest teilweise gleichzeitig auszuführen.

In alternativen, nicht in den Zeichnungsfiguren dargestellten Ausführungsbeispielen werden dieselben Schritte in anderen Reihenfolgen ausgeführt. Dabei sind verschiedene Reihenfolgen möglich, solange sichergestellt ist, dass die miteinander zu verbindenden Teile rechtzeitig bereitgestellt sind, um miteinander verbunden werden zu können.

In alternativen Ausführungsbeispielen stellt das Mittelteil in der fertig hergestellten Tropfkammer keinen Griffring bereit, sondern dient beispielsweise lediglich als Verbindungselement, welches Oberteil und Unterteil zusammenhält.

Beim erfindungsgemäßen Verfahren wird kein Mittelteil in Gestalt eines Griffrings mittels eines aufwendigen Spritzgießverfahrens an der Außenfläche der Tropfkammer angeformt. Anstatt dessen wird auf ein vorgefertigtes Mittelteil zurückgegriffen, welches im Zuge des Verfahrens lediglich mit dem Oberteil und/oder dem Unterteil verbunden werden muss, was den fertigungstechnischen Aufwand der Herstellung einer dreiteiligen Tropfkammer maßgeblich verringert. Die Verringerung des fertigungstechnischen Aufwands ist vorliegend besonders sinnvoll, da es sich bei Infusionssystemen um Massenartikel handelt, die in großer Stückzahl hergestellt werden.

In dem erfindungsgemäßen Verfahren werden entweder das Oberteil und das Unterteil jeweils mit dem Mittelteil verbunden oder direkt miteinander verbunden. In bevorzugten Ausführungsbeispielen der Erfindung erfolgt das Verbinden durch Lösungsmittel-Fügen.

Alternativ zu dem beschriebenen Fügeverfahren des Lösungsmittel-Fügens sind in anderen Ausführungsbeispielen je nach Beschaffenheit der Materialien auch andere Fügeverfahren möglich, beispielsweise Kleben unter Verwendung eines Klebstoffs, beispielsweisebevorzugt UV-Kleben, insbesondere mittels Acrylatkleber, oder Schweißen wie Spiegelschweißen, Torsionsschweißen oder bevorzugt Ultraschallschweißen. In vielen Fällen sind dabei Schweißverfahren bevorzugt, weil sie ohne eine weitere Materialkomponente auskommen.

Die genannten Fügeverfahren (Lösungsmittel-Fügen, Schweißen, Kleben) gehen mit einem geringeren fertigungstechnischen Aufwand einher als das konventionell zum Anformen des Griffrings verwendete Spritzgießverfahren. In der einen beschriebenen Ausführungsform kann das Verbinden des Oberteils mit dem Mittelteil mittels desselben Fügeverfahrens erfolgen wie das Verbinden des Unterteils mit dem Mittelteil. Das Verbinden des Oberteils mit dem Mittelteil kann aber auch mittels eines anderen Fügeverfahrens erfolgen als das Verbinden des Unterteils mit dem Mittelteil. In der anderen beschriebenen Ausführungsform kann das Verbinden des Mittelteils mit dem Oberteil und/oder dem Unterteil mittels desselben Fügeverfahrens erfolgen wie das Verbinden des Unterteils mit dem Oberteil. Das Verbinden des Mittelteils mit dem Oberteil und/oder dem Unterteil kann aber auch mittels eines anderen Fügeverfahrens erfolgen als das Verbinden des Unterteils mit dem Oberteil.

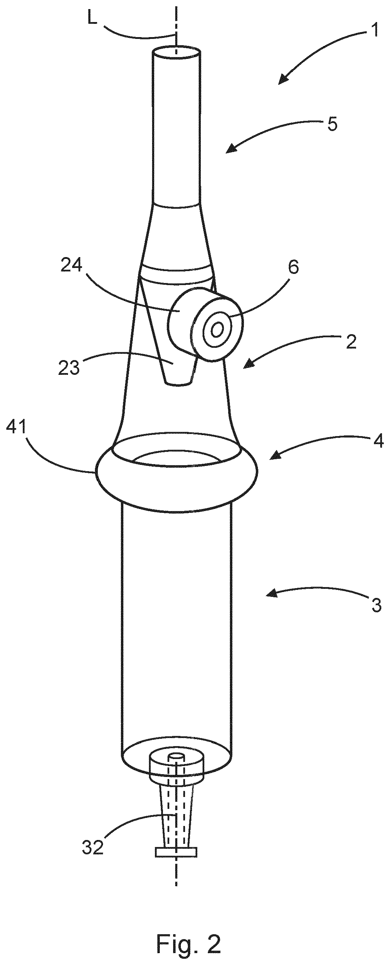

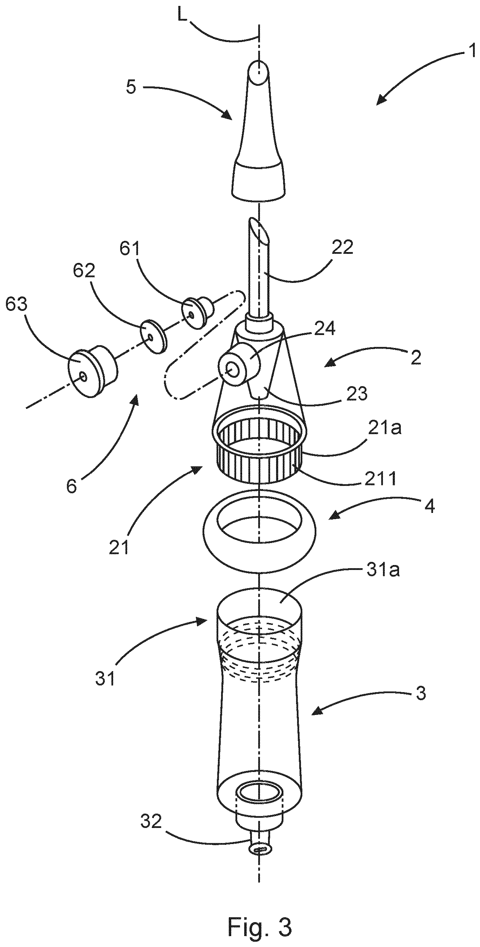

In Fig. 2 und Fig. 3 ist eine Tropfkammer 1 gemäß einem ersten Ausführungsbeispiel dargestellt. Dabei ist die Tropfkammer 1 in ihrer Betriebsstellung mit vertikaler Längsachse L dargestellt. Auf diese Betriebsstellung beziehen sich nachfolgend die Positionsangaben "oben" und "unten". Diese Bezugnahme auf die Betriebsstellung soll die nachfolgende Beschreibung vereinfachen. Der Gegenstand der Erfindung wird dadurch nicht auf eine bestimmte Orientierung der Tropfkammer 1 beschränkt.

Die Tropfkammer 1 umfasst ein Oberteil 2, ein Unterteil 3 und ein Mittelteil 4.

Das Oberteil 2 ist mit seinem unteren Endbereich 21 mit dem Mittelteil 4 verbunden. Im dargestellten Ausführungsbeispiel ist das Mittelteil 4 ringförmig. Um die Verbindung zwischen Oberteil 2 und Mittelteil 4 zu verbessern, können am Oberteil 2 und/oder am Mittelteil 4 Fügestrukturen 211 vorgesehen sein. Beispielhaft ist in Fig. 3 eine geriffelte Struktur 211 im Bereich des unteren Endbereiches 21 des Oberteils 2 dargestellt, welches eine optimale Verklebung ermöglicht.

Das Unterteil 3 ist mit seinem oberen Endbereich 31 mit dem Mittelteil 4 verbunden. Um die Verbindung zwischen Unterteil 3 und Mittelteil 4 zu verbessern, können am Unterteil 3 und Mittelteil 4 Fügestrukturen (nicht dargestellt) vorgesehen sein.

Um die Verbindung zwischen Oberteil 2 bzw. Unterteil 3 und Mittelteil 4 zu verbessern, können Nuten, Wulste oder andere Verbindungsstrukturen (nicht dargestellt) vorgesehen sein. Solche Verbindungsstrukturen können beispielsweise eine formschlüssige Verbindung ermöglichen und/oder die Fügeflächen für eine etwaige Verbindung durch Lösungsmittel-Fügen, Schweißen oder Kleben verbessern.

Indem das Oberteil 2 und das Unterteil 3 jeweils mit dem Mittelteil 4 verbunden sind, ergibt sich zwischen Oberteil 2 und Unterteil 3 über das Mittelteil 4 eine fluiddichte Verbindung. Das heißt, die Tropfkammer 1 bildet einen Hohlkörper, der mit Ausnahme der Anschlüsse und der Belüftungsvorrichtung, die nachfolgend noch beschrieben werden, fluiddicht ist.

Die Innenwand dieses Hohlkörpers setzt sich aus mehreren Innenwandabschnitten zusammen, nämlich zumindest einem ersten Innenwandabschnitt, der zumindest im Wesentlichen der Innenwand des Oberteils 2 entspricht, und einem zweiten Innenwandabschnitt, der zumindest im Wesentlichen der Innenwand des Unterteils 3 entspricht. Daneben kann ein dritter Innenwandabschnitt vorliegen, der von einem Abschnitt des Mittelteils 4 gebildet wird. In anderen Worten: Wenn das Oberteil 2 und das Unterteil 3 nicht auf Stoß zusammentreffen, wenn sie jeweils mit dem Mittelteil 4 verbunden werden, trägt das Mittelteil 4 zur Ausbildung der Wandung der Tropfkammer bei.

An dem oberen Ende des Oberteils 2, das dem unteren Endbereich 21 des Oberteils 2 in vertikaler Längsrichtung L gegenüberliegt, ist ein erster Anschluss 22 vorgesehen. Dieser erste Anschluss 22 ist bestimmt, mit einem in den Zeichnungsfiguren nicht dargestellten Behälter für die dem Patienten zu verabreichende Flüssigkeit verbunden zu werden, sodass die Flüssigkeit aus dem Behälter in die Tropfkammer 1 gelangen kann. In der Darstellung in Fig. 3 handelt es sich bei dem ersten Anschluss 22 um einen hohlen Dorn, mit dem ein Septum, welches den Behälter verschließt, durchstochen werden kann. Ein derartiger hohler Dorn, welcher in seinem Inneren mehrere Kanäle aufweist, wird allgemein als "Spike" bezeichnet. Es ist bevorzugt, den ersten Anschluss 22 mit dem Gehäuseteil des Oberteils 2 monolithisch herzustellen. Das Oberteil 2 besteht dann abgesehen von der Belüftungsvorrichtung 6 aus einem Teil, was fertigungstechnisch vorteilhaft ist und ferner sicherstellt, dass der Anschluss 22 sicher an der Tropfkammer 1 angebracht ist.

In alternativen, nicht in den Zeichnungsfiguren dargestellten Ausführungsbeispielen sind andere Systeme vorgesehen, um die Tropfkammer 1 mit dem Behälter zu verbinden, beispielsweise Kupplungssysteme, welche es nicht zulassen, dass die Tropfkammer und der Behälter, nachdem diese miteinander verbunden worden sind, wieder getrennt werden können. Es ist auch möglich, dass der Anschluss am oberen Ende der Tropfkammer 1 mit dem Behälter bzw. dem Behälterverschluss fest verbunden ist, beispielsweise durch Lösungsmittel-Fügen, Verkleben, Verschweißen oder monolithische Herstellungsweise.

Es kann eine Abdeckkappe 5 vorgesehen sein, mit der der erste Anschluss 22 vor und gegebenenfalls auch nach Verwendung der Tropfkammer 1 abgedeckt wird. Durch das Abdecken wird der Anschluss 22 vor Kontaminationen geschützt. Außerdem wird durch eine ausreichend feste Abdeckung die Arbeitssicherheit erhöht, weil sie Verwender und Patienten vor Stichverletzungen durch einen Spike schützt.

Im oberen Bereich des Oberteils 2 des Inneren der Tropfkammer 1 ist ein Tropfenformer 23 vorgesehen. Flüssigkeit, die aus dem Behälter durch den ersten Anschluss 22 in die Tropfkammer 1 gelangt, tritt über den Tropfenformer 23 in das Innere der Tropfkammer 1 ein. Der Tropfenformer 23 ist so geformt, dass die Flüssigkeit in Form von Tropfen normierter Größe in die Tropfkammer 1 eintritt.

Die Tropfkammer 1 verfügt über eine Belüftungsvorrichtung 6. In den in den Zeichnungsfiguren ist eine Variante dargestellt, bei der das Belüftungsventil 61 in einen im oberen Bereich des Oberteils 2 angeformten Belüftungsstutzen 24 eingepasst ist. In alternativen Ausführungsbeispielen ist statt eines Stutzens eine flache Öffnung in der Wand des Oberteils 2 vorgesehen, in die die Belüftungsvorrichtung eingepasst ist.

Bei dem Belüftungsventil 61 handelt es sich gemäß vorliegendem Ausführungsbeispiel um ein Rückschlagventil, welches Luft über einen Kanal des Anschlusses 22 in den Behälter für die dem Patienten zu verabreichende Flüssigkeit eintreten, nicht aber umgekehrt ein Fluid austreten lässt. Die Funktion des Belüftungsventils 61 besteht beispielsweise darin, dass beim Zusammendrücken des Unterteils 3 Luft in den Behälter gepumpt wird. Durch diese Pumpfunktion kann das Fließen von Flüssigkeit aus dem Behälter in die Tropfkammer initiiert werden. Beim Pumpen wird der mit der Tropfkammer verbundene Schlauch zweckmäßigerweise abgeklemmt, beispielsweise mittels einer für diesen Zweck üblichen Rollenklemme.

Die Belüftungsvorrichtung 6 umfasst neben dem Belüftungsventil 61 ein Belüftungsfilter 62 sowie eine Kappe 63, welche das Belüftungsventil 61 und das Belüftungsfilter 62 um Belüftungsstutzen 24 halten.

Bevorzugt und im Gegensatz zur konventionell üblichen Gestaltung weist das Oberteil 2 eine längliche Form auf, d.h. es seine Länge in Längsrichtung L ist größer als seine Breite in der Richtung quer dazu. Dadurch wird die Ergonomie der Tropfkammer 1 verbessert. Insbesondere wird das Zuspritzen von Arzneimitteln erleichtert, weil der dafür optional vorgesehene Port des Infusionsbehälters für den Anwender besser zugänglich ist, wenn die Tropfkammer 1 im Bereich des Oberteils 2 eine schlanke Form aufweist.

An dem unteren Ende des Unterteils 3, das dem oberen Endbereich 31 des Unterteils 3 in vertikaler Längsrichtung L gegenüberliegt, ist ein zweiter Anschluss 32 vorgesehen. Dieser erste Anschluss 32 ist bestimmt, mit einem Schlauch verbunden zu werden.

Das Mittelteil 4 ist in vorliegendem Ausführungsbeispiel so geformt, dass es nach der Verbindung mit dem Oberteil 2 und dem Unterteil 3 eine Wulst 41 an der Außenfläche der Tropfkammer 1 bereitstellt. Diese Wulst 41 stellt, in Richtung quer zur Längsachse L betrachtet, die breiteste Stelle der Tropfkammer 1 dar. Daher kann die Tropfkammer 1 an der Wulst 41 besonders sicher und bequem angefasst werden, was zu einer sehr ergonomischen und sicheren Handhabbarkeit der Tropfkammer 1 führt. Die Wulst stellt demnach einen Griffring dar.

In einer weiteren Ausgestaltung (b) der erfindungsgemäßen Tropfkammer, die in den Zeichnungen nicht konkret gezeigt ist, die aber anhand der Zeichnung ohne weiteres ableitbar ist, sind der untere Rand 21a des Oberteils und der obere Rand 31a des Unterteils direkt miteinander gefügt, beispielsweise durch Lösungsmittel-Fügen, Verkleben oder Verschweißen. Hier kann das Mittelteil 4 mit einem unteren Endbereich 21 des Oberteils 2 und/oder einem oberen Endbereich 31 des Unterteils 3 gefügt sein, muss es aber nicht, um die Herstellung zu vereinfachen. Auch ohne Fügung mit Oberteil 2 und/oder Unterteil 3 wird das Mittelteil 4 in diesem Fall in der Nut gehalten, die durch den geringeren Durchmesser im Übergang zwischen unterem Endbereich 21 des Oberteils 2 und oberem Endbereich 31 des Unterteils 3 gebildet ist.

Das Oberteil 2 ist zumindest im Wandbereich vorzugsweise aus einem optisch hochwertigen Kunststoff hergestellt, der hochdurchsichtig ist und eine hohe optische Brillanz aufweist. Unter einem hochdurchsichtigen Kunststoff wird dabei ein Kunststoff verstanden, welcher es ermöglicht, dass zumindest ein Abschnitt der Wand des Oberteils 2 eine Lichttransmission im sichtbaren Spektralbereich von mindestens 90% aufweist. Das bedeutet, dass mindestens 90% von auf den Abschnitt der Wand des Oberteils 2 gestrahltes weißes Licht durch den Abschnitt hindurchgeht und somit weniger als 10% reflektiert und absorbiert werden. Bevorzugt ist eine Lichttransmission im sichtbaren Spektralbereich von mindestens 95% aufweist.

Bevorzugt ist die Verwendung von hochdurchsichtigem styrolbasiertem Kunststoff. Bei dem hochdurchsichtigem styrolbasiertem Kunststoff handelt es sich beispielsweise um Polystyrol oder Styrol-Acrylnitril-Copolymer (SAN). Auf diese Weise ist es möglich, die Tropfenbildung am Tropfenformer 23 und den Tropfenabfall hervorragend gut zu beobachten - sei es visuell mit bloßem Auge oder automatisch mittels eines optischen Tropfensensors. In einem konkreten Ausführungsbeispiel besteht das Oberteil 2 mit Ausnahme der Belüftungsvorrichtung 6 aus hochdurchsichtigem Polystyrol. In einem alternativen Ausführungsbeispiel besteht das Oberteil 2 mit Ausnahme der Belüftungsvorrichtung 6 aus hochdurchsichtigem SAN.

Das Unterteil 3 ist zumindest im Wandbereich vorzugsweise aus einem elastischen Kunststoff hergestellt. Auf diese Weise kann die oben beschriebene Pumpfunktion der Tropfkammer 1 optimal bereitgestellt werden. Bevorzugt ist die Verwendung eines styrolbasierten Polymermaterialis mit einem geeigneten und auf die gewünschte Elastizität abgestimmten Grad der Weichheit. Insbesondere bevorzugt ist die Verwendung von Styrol-Butadien-Copolymer (SBC). In dem beschriebenen konkreten Ausführungsbeispiel besteht das Unterteil 3 aus SBC.

Oberteil 2 und Unterteil 3 sind jeweils mit dem Mitteteil 4 verbunden. In bevorzugten Ausführungsbeispielen der Erfindung ist die Verbindung dadurch bereitgestellt, dass die Komponenten miteinander durch Lösungsmittel-Fügen miteinander verbunden sind.

Das Material des Mittelteils 4 wird so gewählt, dass sich auf möglichst einfache Weise eine gute Verbindung zwischen den Komponenten herstellen lässt. In dem beschriebenen konkreten Ausführungsbeispiel besteht das Mittelteil 3 aus einem styrolbasierten Polymermaterial, welches sich durch Lösungsmittel-Fügen sowohl mit dem hochdurchsichtigen Polystyrol des Oberteils 2 als auch mit dem elastischen Polystyrol des Unterteils 3 sicher verbinden lässt. Das Material des Mittelteils 4 ist somit sowohl auf das Material des Oberteils 2 als auch auf das Material des Unterteils 3 abgestimmt. Bevorzugt ist für das Mittelteil die Verwendung eines styrolbasierten Polymermaterialis mit einem geeigneten und auf die gewünschte Griffigkeit der Wulst (Griffring) abgestimmten Grad der Weichheit. Insbesondere wird in Betracht gezogen, dass es sich bei dem styrolbasierten Polymermaterial des Mittelteils ebenfalls um SBC handelt.

Weichere Kunststoffe erfüllen in der Regel nicht die Anforderungen, die hinsichtlich der Durchsichtigkeit und optischen Brillanz an das Oberteil 2 einer Tropfkammer 1 gestellt werden. Hochdurchsichtige Kunststoffe erfüllen in der Regel nicht die mechanischen Anforderungen, die im Hinblick auf die Pumpbarkeit an das Unterteil 3 einer Tropfkammer 1 gestellt werden. Die Erfindung ermöglicht eine Vielzahl an Kombinationen der Materialien für Oberteil 2 und Unterteil 3, die jeweils hinsichtlich der optischen bzw. mechanischen Eigenschaften gut geeignet sind und die weiteren Anforderungen wie etwa chemische Beständigkeit und Langlebigkeit erfüllen.

Die Handhabbarkeit und Sicherheit der Tropfkammer 1 ist in weiteren Ausführungsbeispielen der Erfindung dadurch weiter verbessert, dass das Mittelteil nicht nur eine exponierte Stelle wie etwa eine Wulst o.ä. an der Außenwand der Tropfkammer 1 bereitstellt, sondern zusätzlich aus einem Material besteht, das sich besonders angenehm und sicher mit der Hand greifen lässt. Dafür haben sich weiche Materialien und/oder Materialien mit einer guten Griffigkeit wie beispielsweise SBC als besonders geeignet erwiesen. Es ist dabei nicht erforderlich, dass das Mittelteil 4 insgesamt aus einem derartigen Material gefertigt ist. Vielmehr ist es ausreichend, wenn die Abschnitte, an denen die Tropfkammer 1 angefasst und gehalten werden soll, aus einem derartigen Material bestehen. In speziellen Ausführungsbeispielen der Erfindung ist das Mittelteil 4 daher aus verschiedenen Materialien zusammengesetzt, wobei an den Stellen, an denen die Verbindung mit dem Oberteil 2 und dem Unterteil 3 hergestellt wird, ein für eine besonders einfach herzustellende und/oder besonders feste Verbindung optimiertes Material vorliegt, und an den Stellen, an denen die Tropfkammer 1 angefasst und gehalten werden soll, ein für das Greifen optimiertes Material vorliegt. Insbesondere ist es dabei möglich und vorteilhaft, wenn das Mittelteil 4 an den Stellen, an denen die Tropfkammer 1 angefasst und gehalten werden soll, ein weiches, griffiges Material aufweist, und ansonsten aus einem härteren Material besteht, um der Tropfkammer 1 zusätzliche mechanische Stabilität zu verleihen.

Alternativ zu dem beschriebenen Fügeverfahren des Lösungsmittel-Fügens sind in anderen Ausführungsbeispielen je nach Beschaffenheit der Materialien auch andere Fügeverfahren möglich, beispielsweise Kleben unter Verwendung eines Klebstoffs, bevorzugt UV-Kleben, insbesondere mittels Acrylatkleber, oder Schweißen wie Spiegelschweißen, Torsionsschweißen oder bevorzugt Ultraschallschweißen. In vielen Fällen sind dabei Schweißverfahren bevorzugt, weil sie ohne eine weitere Materialkomponente auskommen. Auch für die alternativen Fügeverfahren müssen die Materialeigenschaften der Fügekomponenten aufeinander abgestimmt sein, sodass die Erfindung auch im Zusammenhang mit den alternativen Fügeverfahren vorteilhaft ist. Insbesondere ist es vorteilhaft, dass die genannten Fügeverfahren mit einem geringeren fertigungstechnischen Aufwand einhergehen als das konventionell zum Anformen des Griffrings verwendete Spritzgießverfahren. Das Verbinden des Oberteils 2 mit dem Mittelteil 4 kann mittels desselben Fügeverfahrens erfolgen wie das Verbinden des Unterteils 3 mit dem Mittelteil 4. Das Verbinden des Oberteils 2 mit dem Mittelteil 4 kann auch mittels eines anderen Fügeverfahrens erfolgen als das Verbinden des Unterteils 3 mit dem Mittelteil 4.

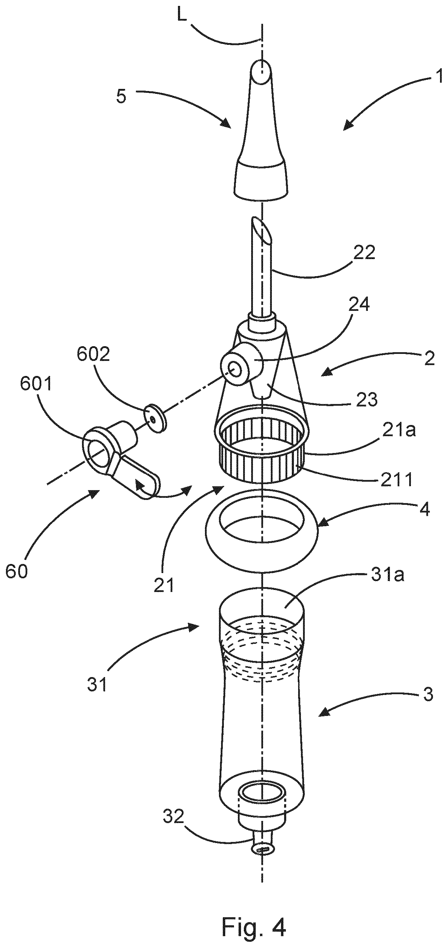

Fig. 4 zeigt eine schematische Ansicht der Tropfkammer 11 gemäß einem zweiten Ausführungsbeispiel in einer Explosionsdarstellung. Die Tropfkammer 11 gemäß dem zweiten Ausführungsbeispiel unterscheidet sich von der Tropfkammer 1 gemäß dem ersten Ausführungsbeispiel nur hinsichtlich der Belüftungsvorrichtung. Die übrigen Elemente entsprechen einander und sind mit gleichen Bezugszeichen gekennzeichnet. Eine nähere Beschreibung der übrigen Elemente und ihrer Funktion erübrigt sich daher.

Die Belüftungsvorrichtung 60 der Tropfkammer 11 gemäß des zweiten Ausführungsbeispiels umfasst neben dem Belüftungsventil 601 ein Belüftungsfilter 602.

Bei dem Belüftungsventil 601 handelt es sich um eine Klappe, welche manuell geöffnet und geschlossen werden kann. Die Funktion der Belüftungsvorrichtung 60 (zweites Ausführungsbeispiel) entspricht der obenstehend beschriebenen Funktion der Belüftungsvorrichtung 6 (erstes Ausführungsbeispiel).

Die äußere Grundform des Oberteils ist in den dargestellten Ausführungsbeispielen konisch. In alternativen, nicht in den Zeichnungsfiguren dargestellten Ausführungsbeispielen kann das Oberteil auch eine andere, beispielsweise zylindrische oder kuppelförmige Grundform aufweisen.

Die äußere Grundform des Unterteils ist in den dargestellten Ausführungsbeispielen zylindrisch. In alternativen, nicht in den Zeichnungsfiguren dargestellten Ausführungsbeispielen kann das Unterteil auch eine andere, beispielsweise konische Grundform aufweisen.

Die Tropfkammer weist in den dargestellten Ausführungsbeispielen abgesehen von der Belüftungsvorrichtung Rotationssymmetrie bezüglich einer Rotation um die Längsachse L auf. In alternativen, nicht in den Zeichnungsfiguren dargestellten Ausführungsbeispielen kann die Tropfkammer auch nicht rotationssymmetrisch sein, beispielsweise elliptische Querschnitte aufweisen.

Das Mittelteil 4 der erfindungsgemäßen Tropfkammer 1 ist als Muffe ausgebildet, die für die fluiddichte Verbindung von Oberteil 2 und Unterteil 3 sorgt. Dabei ist es möglich, dass das Oberteil 2 und das Unterteil 3 jeweils so mit dem Mittelteil 4 verbunden sind, dass das Oberteil 2 bzw. dessen unterer Rand 21a und das Unterteil 3 bzw. dessen oberer Rand 31a voneinander beanstandet sind und zwischen Oberteil 2 und Unterteil 3 beispielweise ein umlaufender Spalt vorliegt. In diesem Fall trägt das Mittelteil 4, über das das Oberteil 2 und das Unterteil 3 miteinander verbunden sind, zur Ausbildung der Innenwandung der Tropfkammer 1 bei. Das Oberteil 2 und das Unterteil 3 können aber auch so angeordnet sein, dass der untere Rand 21a des Oberteils 2 und der obere Rand 31a des Unterteils 3 auf Stoß liegen, d.h. Oberteil 2 und Unterteil 3 sind dann nicht voneinander beabstandet sondern berühren einander. Dem Mittelteil 4 kommt dann die Funktion zu, das Oberteil 2 und das Unterteil 3 in dieser Lage relativ zueinander festzuhalten und gegebenenfalls die Verbindung zwischen Oberteil 2 und Unterteil 3 weiter abzudichten. In diesem Fall trägt das Mittelteil 4 nicht zur Ausbildung der Innenwandung der Tropfkammer 1 bei.

Zusammenfassend kann eine Ausführungsform der Erfindung folgendermaßen beschrieben werden: Verfahren zum Herstellen einer Tropfkammer 1, umfassend die Schritte des Bereitstellens eines Oberteils 2, eines Unterteils 3 und eines Mittelteils (4) und des Verbindens des Oberteils 2, des Unterteils 3 und des Mittelteils 4. Das Verbinden erfolgt so, dass das Oberteil 2 und das Unterteil 3 über das Mittelteil 4 fluiddicht miteinander verbunden sind, oder so, dass das Oberteil 2 und das Unterteil 3 direkt fluiddicht miteinander verbunden sind und das Mittelteil 4 außen an dem Oberteil 2 und/oder dem Unterteil 3 angebracht ist.

Erfindungsgemäß wird so beispielsweise eine Tropfkammer 1 erhalten, die neben dem Oberteil 2 und dem Unterteil 3 ein vorgefertigtes Mittelteil 4 aufweist, welches so eingerichtet ist, dass es eine jeweils unmittelbare fluiddichte Verbindung mit dem Oberteil und mit dem Unterteil schafft, indem ein unterer Endbereich 21 des Oberteils 2 und ein oberer Endbereich 31 des Unterteils 3 jeweils mit dem Mittelteil 4 gefügt sind, oder welches so eingerichtet ist, dass es eine Überbrückung eines unteren Endbereichs 21 des Oberteils 2 und eines oberen Endbereichs 31 des Unterteils 3 bereitstellt, wobei der untere Rand 21a des Oberteils und der obere Rand 31a des Unterteils direkt miteinander gefügt sind und das Mittelteil 4 die Überbrückung bereitstellt.

## Patentansprüche

1. Verfahren zum Herstellen einer Tropfkammer (1), umfassend die Schritte:
A) Bereitstellen eines Oberteils (2) für die Tropfkammer (1) mit einem ersten Anschluss, welcher bestimmt ist, mit einem Behälter verbunden zu werden,
B) Bereitstellen eines Unterteils (3) für die Tropfkammer (1) mit einem zweiten Anschluss (32), welcher mit einem Schlauch verbunden ist oder welcher bestimmt ist, mit einem Schlauch verbunden zu werden, und
C) Bereitstellen eines Mittelteils (4) für die Tropfkammer (1), das als Muffe ausgebildet ist,
D) Verbinden des Oberteils (2), des Unterteils (3) und der Muffe,
sodass das Oberteil (2) und das Unterteil (3) über die Muffe fluiddicht miteinander verbunden sind,
wobei das Oberteil (2) und die Muffe durch Lösungsmittel-Fügen miteinander verbunden sind, und
wobei das Unterteil (3) und die Muffe durch Lösungsmittel-Fügen miteinander verbunden sind.

2. Tropfkammer (1) für ein Infusions- oder Transfusionssystem, insbesondere hergestellt durch ein Verfahren gemäß Anspruch 1, wobei die Tropfkammer (1) umfasst:
ein Oberteil (2) mit einem ersten Anschluss, welcher bestimmt ist, mit einem Behälter verbunden zu werden,
ein Unterteil (3) mit einem zweiten Anschluss (32), welcher mit einem Schlauch verbunden ist oder welcher bestimmt ist, mit einem Schlauch verbunden zu werden, und
ein vorgefertigtes Mittelteil (4), welches als Muffe ausgebildet und so eingerichtet ist, dass es eine jeweils unmittelbare fluiddichte Verbindung mit dem Oberteil und mit dem Unterteil schafft, indem ein unterer Endbereich (21) des Oberteils (2) und ein oberer Endbereich (31) des Unterteils (3) jeweils mit dem Mittelteil (4) gefügt sind,
wobei das Oberteil (2) und das Mittelteil (4) durch Lösungsmittel-Fügen miteinander verbunden sind, und
wobei das Unterteil (3) und das Mittelteil (4) durch Lösungsmittel-Fügen miteinander verbunden sind.

3. Tropfkammer (1) gemäß Anspruch 2,
wobei das Oberteil (2) ein Polymermaterial umfasst,
wobei das Polymermaterial bevorzugt ein styrolbasiertes Polymermaterial ist,
wobei das styrolbasierte Polymermaterial mehr bevorzugt ein Polystyrolmaterial oder ein Styrol-Acrylnitril-Copolymer-Material, insbesondere ein hochdurchsichtiges Polystyrolmaterial oder ein hochdurchsichtiges Styrol-Acrylnitril-Copolymer-Material ist.

4. Tropfkammer (1) gemäß einem der Ansprüche 2 bis 3,
wobei das Unterteil (3) ein Polymermaterial umfasst,
wobei das Polymermaterial bevorzugt ein styrolbasiertes Polymermaterial ist,
wobei das styrolbasierte Polymermaterial mehr bevorzugt ein Styrol-Butadien-Copolymer-Material ist.

5. Tropfkammer (1) gemäß einem der Ansprüche 2 bis 4,
wobei das Mittelteil (4) ein Polymermaterial umfasst,
wobei das Polymermaterial bevorzugt ein styrolbasiertes Polymermaterial ist,
wobei das styrolbasierte Polymermaterial mehr bevorzugt ein Styrol-Butadien-Copolymer-Material ist.

6. Tropfkammer (1) gemäß einem der Ansprüche 2 bis 5,
wobei zumindest ein Abschnitt einer Wand des Oberteils (2) eine Lichttransmission im sichtbaren Spektralbereich von mindestens 90%, bevorzugt mindestens 95% aufweist.

7. Tropfkammer (1) gemäß einem der Ansprüche 2 bis 6,
wobei das Mittelteil (4) an einer Außenfläche der Tropfkammer (1) eine Wulst (41) bereitstellt.

8. Tropfkammer (1) gemäß einem der Ansprüche 2 bis 7,
wobei der erste Anschluss (22) als Stechvorrichtung, insbesondere als hohle Stechvorrichtung zum Durchstechen der Wand eines Behälter für eine einem Patienten zu verabreichende Flüssigkeit oder eines am Behälter vorgesehenen Septums, ausgebildet ist und/oder
wobei der erste Anschluss (22) mit dem Behälter unlösbar verbunden oder mit dem Behälter unlösbar verbindbar ist.

9. Tropfkammer (1) gemäß einem der Ansprüche 2 bis 8,
wobei der zweite Anschluss (32) als Kupplungsvorrichtung ausgebildet ist, welche mit einer komplementären Kupplungsvorrichtung am Ende eines Schlauchs so verbunden werden kann, dass eine dichte Fluidverbindung zwischen dem Inneren der Tropfkammer (1) und dem Schlauch besteht,
wobei die Kupplungsvorrichtungen bevorzugt trocken abschließen, wenn sie nicht miteinander verbunden sind.

10. Tropfkammer (1) gemäß einem der Ansprüche 2 bis 9,
wobei das Oberteil (2) einen ersten Anschluss (22), welcher mit einem Behälter verbunden ist oder welcher bestimmt ist, mit einem Behälter verbunden zu werden, aufweist, und
wobei das Unterteil (3) einen zweiten Anschluss, welcher mit einem Schlauch verbunden ist oder welcher bestimmt ist, mit einem Schlauch verbunden zu werden, aufweist.

11. Infusions- oder Transfusionssystem, welches eine Tropfkammer (1) gemäß einem der Ansprüche 2 bis 10 umfasst.

## Claims

1. A method of manufacturing a drip chamber (1), comprising the steps of:
A) providing an upper part (2) for the drip chamber (1) with a first connection intended to be connected to a container,
B) providing a lower part (3) for the drip chamber (1) with a second connection (32) connected to a tube or intended to be connected to a tube, and
C) providing a middle part (4) for the drip chamber (1), which part is designed as a sleeve,
D) connecting the upper part (2), the lower part (3) and the sleeve,
such that the upper part (2) and the lower part (3) are connected to each other in a fluid-tight manner via the sleeve,
wherein the upper part (2) and the sleeve are connected to each other by solvent joining, and
wherein the lower part (3) and the sleeve are connected to each other by solvent joining.

2. A drip chamber (1) for an infusion or transfusion system, in particular manufactured by a method according to claim 1, wherein the drip chamber (1) comprises:
an upper part (2) with a first connection intended to be connected to a container,
a lower part (3) with a second connection (32) connected to a tube or intended to be connected to a tube, and
a prefabricated middle part (4), designed as a sleeve and configured to provide a direct fluid-tight connection with the upper part and with the lower part, respectively, by joining a lower end region (21) of the upper part (2) and an upper end region (31) of the lower part (3) to the middle part (4), respectively,
wherein the upper part (2) and the middle part (4) are connected to each other by solvent joining, and
wherein the lower part (3) and the middle part (4) are connected to each other by solvent joining.

3. The drip chamber (1) according to claim 2,
wherein the upper part (2) comprises a polymer material,
wherein the polymer material is preferably a styrene-based polymer material,
wherein the styrene-based polymer material is more preferably a polystyrene material or a styrene-acrylonitrile copolymer material, in particular a highly transparent polystyrene material or a highly transparent styrene-acrylonitrile copolymer material.

4. The drip chamber (1) according to any one of claims 2 to 3,
wherein the lower part (3) comprises a polymer material,
wherein the polymer material is preferably a styrene-based polymer material,
wherein the styrene-based polymer material is more preferably a styrenebutadiene copolymer material.

5. The drip chamber (1) according to any one of claims 2 to 4,
wherein the middle part (4) comprises a polymer material,
wherein the polymer material is preferably a styrene-based polymer material,
wherein the styrene-based polymer material is more preferably a styrenebutadiene copolymer material.

6. The drip chamber (1) according to any one of claims 2 to 5,
wherein at least a portion of a wall of the upper part (2) has a light transmission in the visible spectral range of at least 90%, preferably at least 95%.

7. The drip chamber (1) according to any one of claims 2 to 6,
wherein the middle part (4) provides a protrusion (41) on an outer surface of the drip chamber (1).

8. The drip chamber (1) according to any one of claims 2 to 7,
wherein the first connection (22) is formed as a piercing device, in particular as a hollow piercing device, for piercing the wall of a container for a liquid to be administered to a patient or of a septum provided at the container, and/or
wherein the first connection (22) is non-detachably connected to the container or is non-detachably connectable to the container.

9. The drip chamber (1) according to any one of claims 2 to 8,
wherein the second connection (32) is formed as a coupling device which may be connected to a complementary coupling device at the end of a tube such that a tight fluid connection exists between the interior of the drip chamber (1) and the tube,
wherein the coupling devices are preferably closed in a dry manner when not connected to each other.

10. The drip chamber (1) according to any one of claims 2 to 9,
wherein the upper part (2) has a first connection (22) connected to a container or intended to be connected to a container, and
wherein the lower part (3) has a second connection connected to a tube or intended to be connected to a tube.

11. An infusion or transfusion system comprising a drip chamber (1) according to any one of claims 2 to 10.

## Revendications

1. Procédé de fabrication d'une chambre compte-gouttes (1), comprenant les étapes consistant à :
A) fournir une partie supérieure (2) pour la chambre compte-gouttes (1) comportant un premier raccord destiné à être raccordé à un récipient,
B) fournir une partie inférieure (3) pour la chambre compte-gouttes (1) comportant un second raccord (32) qui est raccordé à un tuyau ou qui est destiné à être raccordé à un tuyau, et
C) fournir une partie centrale (4) pour la chambre compte-gouttes (1) qui est réalisée sous forme de manchon,
D) relier la partie supérieure (2), la partie inférieure (3) et le manchon,
de sorte que la partie supérieure (2) et la partie inférieure (3) sont reliées l'une à l'autre de manière étanche aux fluides par l'intermédiaire du manchon,
dans lequel la partie supérieure (2) et le manchon sont reliés l'un à l'autre par assemblage par solvant, et
dans lequel la partie inférieure (3) et le manchon sont reliés l'un à l'autre par assemblage par solvant.

2. Chambre compte-gouttes (1) pour un système de perfusion ou de transfusion, en particulier fabriquée par un procédé selon la revendication 1, dans laquelle la chambre compte-gouttes (1) comprend :
une partie supérieure (2) comportant un premier raccord qui est destiné à être raccordé à un récipient,
une partie inférieure (3) comportant un second raccord (32) qui est raccordé à un tuyau ou qui est destiné à être raccordé à un tuyau, et
une partie centrale (4) préfabriquée qui est réalisée sous forme de manchon et est conçue de sorte qu'elle crée une liaison directe étanche aux fluides respectivement avec la partie supérieure et avec la partie inférieure, dans laquelle une zone d'extrémité inférieure (21) de la partie supérieure (2) et une zone d'extrémité supérieure (31) de la partie inférieure (3) sont assemblées respectivement avec la partie centrale (4),
dans laquelle la partie supérieure (2) et la partie centrale (4) sont reliées l'une à l'autre par assemblage par solvant, et
dans laquelle la partie inférieure (3) et la partie centrale (4) sont reliées l'une à l'autre par assemblage par solvant.

3. Chambre compte-gouttes (1) selon la revendication 2,
dans laquelle la partie supérieure (2) comprend un matériau polymère,
dans laquelle le matériau polymère est de préférence un matériau polymère à base de styrène,
dans laquelle le matériau polymère à base de styrène est plus préférentiellement un matériau polystyrène ou un matériau copolymère styrène-acrylonitrile, en particulier un matériau polystyrène hautement transparent ou un matériau copolymère styrène-acrylonitrile hautement transparent.

4. Chambre compte-gouttes (1) selon l'une des revendications 2 à 3,
dans laquelle la partie inférieure (3) comprend un matériau polymère,
dans laquelle le matériau polymère est de préférence un matériau polymère à base de styrène,
dans laquelle le matériau polymère à base de styrène est plus préférentiellement un matériau copolymère styrène-butadiène.

5. Chambre compte-gouttes (1) selon l'une des revendications 2 à 4,
dans laquelle la partie centrale (4) comprend un matériau polymère,
dans laquelle le matériau polymère est de préférence un matériau polymère à base de styrène,
dans laquelle le matériau polymère à base de styrène est plus préférentiellement un matériau copolymère styrène-butadiène.

6. Chambre compte-gouttes (1) selon l'une des revendications 2 à 5,
dans laquelle au moins une section d'une paroi de la partie supérieure (2) présente une transmission de lumière dans le domaine spectral visible d'au moins 90 %, de préférence d'au moins 95 %.

7. Chambre compte-gouttes (1) selon l'une des revendications 2 à 6,
dans laquelle la partie centrale (4) fournit un bourrelet (41) sur une surface extérieure de la chambre compte-gouttes (1).

8. Chambre compte-gouttes (1) selon l'une des revendications 2 à 7,
dans laquelle le premier raccord (22) est réalisé sous forme de dispositif de perçage, en particulier sous forme de dispositif de perçage creux pour le perçage de la paroi d'un récipient pour un liquide à administrer à un patient ou d'un septum prévu sur le récipient, et/ou
dans laquelle le premier raccord (22) est raccordé de manière inamovible au récipient ou peut être raccordé de manière inamovible au récipient.

9. Chambre compte-gouttes (1) selon l'une des revendications 2 à 8,
dans laquelle le second raccord (32) est réalisé sous forme de dispositif d'accouplement qui peut être raccordé à un dispositif d'accouplement complémentaire au niveau de l'extrémité d'un tuyau de sorte qu'un raccordement fluidique étanche existe entre l'intérieur de la chambre compte-gouttes (1) et le tuyau,
dans laquelle les dispositifs d'accouplement se ferment de préférence à sec lorsqu'ils ne sont pas raccordés l'un à l'autre.

10. Chambre compte-gouttes (1) selon l'une des revendications 2 à 9,
dans laquelle la partie supérieure (2) présente un premier raccord (22) qui est raccordé à un récipient ou qui est destiné à être raccordé à un récipient, et
dans laquelle la partie inférieure (3) présente un second raccord qui est raccordé à un tuyau ou qui est destiné à être raccordé à un tuyau.

11. Système de perfusion ou de transfusion comprenant une chambre compte-gouttes (1) selon l'une des revendications 2 à 10.
